# EUROPEAN PATENT APPLICATION

(11) **EP 4 285 888 A1**
(43) Date of publication of application: **06.12.2023**
(21) Application number: 22176620.7
(22) Date of filing: 01.06.2022
(51) Int. Cl.: A61K 6/30, A61K 6/61, A61K 6/62, A61K 6/77, A61K 6/78, A61K 6/887

(54) **COMPOSITE MATERIAL HAVING REDUCED POROSITY AND A PROCESS FOR PRODUCING THEREOF**

(71) Applicant: DENTSPLY DETREY GmbH, 78467 Konstanz (DE)
(72) Inventor: Elsner, Oliver, 78467 Konstanz (DE); Brugger, Stefan, 78467 Konstanz (DE)
(74) Representative: Pichova, Vanda

(57) **Abstract**

The present invention relates to a process for producing a composite material with reduced porosity by applying a vacuum to the mixing container before actual mixing steps. The composite material comprises a monomer mixture and a glass filler or a blend of glass fillers. The monomer mixture comprises at least one polymerizable monomer, a polymerization initiator and a polymerization stabilizer. Optionally, the composite material may comprise at least one pigment. The porosity of the composite material was determined and the porosity was compared with the porosity of the composite material produced by a process wherein a vacuum was not applied before all of the mixing steps. The preferred mixing container of the processes of the present invention is a planetary mixer or a centrifugal mixer. The composite material is for use in the dental industry.

## Description

### BACKGROUND

The present invention relates to a dental composite material used for a filling restoration, a prosthetic restoration, a temporary sealing, a temporary adhering, a prosthesis preparation, an adhesion, a cementation, a crypt fissure sealing in the dental field, more specifically relates to pasty composite material prepared by mixing a filler and a polymerizable monomer, which is used for a composite resin, a temporary sealing material, a resin cement, an adhesive or a fissure sealant.

In the dental field, a composite material is prepared by a process comprising kneading and mixing a filler and at least one polymerizable monomer in the form of a paste. The pasty composite material (the composite material in the form of a paste) is provided to dentists and dental technicians, which are the users of the composite material, in a packaging container filled with the composite material. Although the composite material may be blended with an adhesive monomer, a pigment, or the like, in accordance with the purpose of use, the composite material is generally prepared in the form of a paste by mixing a filler and at least one polymerizable monomer in the appropriate amount, and then is filled in a packaging container.

In the course of the mixing and kneading steps of manufacturing the paste, air is included into the material which causes its porosity. Porosity and air inclusions (bubbles) effect adversely the mechanical and esthetic properties of the dental composite material.

EP 2 985016 and US 10,780,027 of Shofu, Inc. disclose a pasty composite material including a silanated filler and a mixed polymerizable monomer, having a stable pasty property and a process for producing thereof. Mixing and kneading steps of the process are carried out under normal pressure, being followed by a vacuum-mixing step in order to reduce the air inclusions and thus porosity of the paste. In the said vacuum-mixing steps, the material is first mixed and then a vacuum is applied. In other words, the paste is degassed after mixing. A presence of bubbles in the composite material was tested by a Bubble Mixing Test. The results were 'B'. It is an object of the present invention to provide a composite material with a lower level of porosity when compared with the prior art.

### BRIEF SUMMARY

**Composite material** in the terms of this invention is a pasty composite material
**Composite material** in the terms of this invention is either an unshaded composite material or a shaded composite material
A **shaded composite material** in the terms of this invention is a composite material comprising at least one pigment
rpm in the terms of this invention is a unit of the impeller speed (revolutions per minute).
**Speed or Mixing speed** in the terms of this invention is an impeller speed
**Ormosil- Resin** in the terms of this invention is a mixture of ethoxylated bisphenol dimethacrylate, dodecanediol dimethacrylate and Ormocer^{®}, which is a well known product in the dental field.

Dentists and dental technicians are requesting a composite material having reduced porosity. Reduced porosity leads to more uniform polymerization and to the increased quality of the final product. Reduced porosity has also profound esthetic and optical advantages; the need to polish the composite material is limited. In addition to this, reduced porosity decreases likelihood of cracks and weakness of the composite material which increases clinical service time. Furthermore, reduced porosity increases thermal stability of the composite material.

It is an object of the present invention to provide a composite material with a lower level of porosity and a process for producing thereof.

The embodiments described herein can prepare a composite material having a reduced porosity by that the mixing steps are carried out under a vacuum in order to reduce air inclusions. The vacuum is applied before the actual mixing of the single components starts.

### BRIEF DESCRIPTION OF THE FIGURES

Objects, features, and advantages of the present invention will also become apparent upon reading the following description in conjunction with the figures, in which:
**Figs. 1** **(****1a** **and** **1b****)** exhibit a process for producing a composite material in accordance with embodiments of the present invention, wherein a vacuum is applied into the mixing container before every single mixing step starts
**Figs. 2** **(****2a** **and** **2b****)** exhibit a process for producing a composite material, wherein a vacuum is applied into the mixing container just before the last step or before the last two steps start
**Figs.3** **(****3a****,** **3b** **and** **3c****)** exhibit results of the porosity test of the composite material of this invention and of the comparative material

### DETAILED DESCRIPTION

Described herein is a composite material comprising a glass filler or a blend of glass fillers, a monomer mixture and optionally at last one pigment, wherein the monomer mixture comprises one or more polymerizable monomers, a polymerization initiator and a polymerization stabilizer; the composite material is produced by the following process:
Mixing a glass filler or a blend of glass fillers and a monomer mixture and optionally at least one pigment in more steps under a vacuum in a mixing container, wherein a vacuum is applied into the mixing container before every single mixing step starts and wherein the vacuum is preferably of 45-800 mbar.

The weight ratio of the monomer mixture and the glass filler or the blend of glass fillers is preferably 2-4 to 5-7, more preferably 3-4 to 5.5-6.5 and most preferably 3.5 to 5.9.

More particularly, the invention describes a process for producing a composite material comprising the steps of:
a. Charging a monomer mixture into the mixing container
b. Charging a part of the glass filler or of the blend of glass fillers into the mixing container to create a mixture, setting the mixture under a vacuum and mixing the mixture
c. Charging a part of the glass filler or of the blend of glass fillers into the mixing container to create a mixture, setting the mixture under a vacuum and mixing the mixture
d. Setting the mixture under a vacuum and mixing it to create a paste
e. Conditioning the paste
f. Setting the paste under a vacuum and mixing it to produce a composite material and optionally
g. Adding at least one pigment and optionally
a glass filler or a blend of glass fillers and/or a monomer mixture to the composite material to create a mixture, setting the mixture under a vacuum and mixing the mixture to produce a composite material.

In another embodiment of the present invention, the step g. of the above described process is as follows:
Adding at least one pigment and a glass filler or a blend of glass fillers and a monomer mixture to the composite material to create a mixture, setting the mixture under a vacuum and mixing the mixture to produce a composite material.

In another embodiment of the present invention there is a process for producing a composite material as described above, wherein:
- the vacuum of steps b., c. and d. is of 650-800 mbar,
- the mixing of step b. is carried out at the speed of 8-12rpm for 2-5 minutes and then at the speed of 13-16 rpm for 8-12 minutes at the temperature of 23-27°C, whereby the step can be carried out 1-5x
- the mixing of step c. is carried out at the speed of 12-16 rpm for 22-27 minutes at the temperature of 23-27°C, whereby the step can be carried out 1-4x
- the mixing of step d. is carried out at the speed of 10-14rpm for 110-130 minutes at the temperature of 23-27°C, whereby this step can be carried out 1-3x
- the conditioning of step e. is carried out at the temperature of 45-55°C for 36-50 hours under anaerobic conditions
- the vacuum of step f. is a vacuum of 210-230 mbar and the mixing of step f. is carried out at the speed of 6-10rpm for 18-22 minutes at the temperature of 46-48°C
- the mixing of step g. is carried out in at least three steps at the temperature of 23-27°C at the speed of 5-15rpm, whereby each of the steps lasts from 5 to 45 minutes and whereby a vacuum of 45-800 mbar is set before every single mixing step starts

In yet another embodiment of the present invention, there is a process for producing a composite material comprising the steps of:
a. Charging a monomer mixture into the mixing container
b. Charging the first part of the glass filler or of the blend of glass fillers into the mixing container to create a mixture, setting the mixture under a vacuum of 700mbar and mixing it at the speed of 10rpm for 3 minutes and then at the speed of 15 rpm for 10mins at the temperature of 25°C.
c. Charging the second part of the glass filler or of the blend of glass fillers into the mixing container to create a mixture, setting the mixture under a vacuum of 700mbar and mixing it at the speed of 10rpm for 3 minutes and then at the speed of 15 rpm for 10mins at the temperature of 25°C.
d. Charging the third part of the of glass filler or of the blend of glass fillers into a mixing container, to create a mixture, setting the mixture under a vacuum of 700mbar and mixing it at the speed of 10rpm for 3 minutes and then at the speed of 15 rpm for 10mins at the temperature of 25 °C.
e. Charging the fourth part of the glass filler or of the blend of glass fillers into a mixing container to create a mixture, setting the mixture under a vacuum of 700mbar and mixing it at the speed of 15rpm for 25 minutes at the temperature of 25°C
f. Charging the fifth part of the glass filler or of the blend of glass fillers into a mixing container to create a mixture, setting the mixture under a vacuum of 700mbar and mixing it at the speed of 15rpm for 25 minutes at the temperature of 25°C.
g. Charging the sixth part of the glass filler or of the blend of glass fillers into a mixing container to create a mixture, setting the mixture under a vacuum of 700mbar and mixing it at the speed of 15rpm for 25 minutes at the temperature of 25 °C to create a mixture
h. Setting the mixture under a vacuum of 700mbar and mixing it at the speed of 12 rpm for 120 minutes at the temperature of 25 °C to create a mixture
i. Setting the mixture under a vacuum of 700 mbar and mixing it at the speed of 12 rpm for 120 minutes at the temperature of 25 °C to create a paste.
j. Conditioning the paste at the temperature of 50 °C for 36-48 hours under anaerobic conditions.
k. Setting the paste under a vacuum of 220 mbar into the mixing container and mixing it at the speed of 8rpm for 20 minutes at the temperature of 48°C to produce a composite material and optionally
l. Adding at least one pigment and optionally a glass filler or a blend of glass fillers and/or a monomer mixture to the composite material to create a mixture, setting the mixture under a vacuum of 700mbar and mixing it at the speed of 15 rpm for 30 minutes at the temperature of 25°C.
m. Setting the mixture under a vacuum of 700 mbar and mixing it at the speed of 15 rpm for 45 minutes at the temperature of 25°C
n. Setting the mixture under a vacuum of 700 mbar and mixing it at the speed of 15 rpm for 45 minutes at the temperature of 25°C
o. Setting the mixture under a vacuum of 700 mbar and mixing it at the speed of 15 rpm for 45 minutes at the temperature of 25°C
p. Setting the mixture under a vacuum of 50 mbar and mixing it at the speed of 8 rpm for 15 minutes at the temperature of 25°C
q. Setting the mixture under a vacuum of 50 mbar and mixing it at the speed of 5 rpm for 5 minutes at the temperature of 25°C to produce a composite material.

In another embodiment of the present invention, the step 1. of the above described process is as follows:
Adding at least one pigment and a glass filler or a blend of glass fillers and a monomer mixture to the composite material to create a mixture, setting the mixture under a vacuum of 700mbar and mixing it at the speed of 15 rpm for 30 minutes at the temperature of 25°C.

The preferred mixing container of the above described processes is a planetary mixer or a centrifugal mixer.

In another embodiment of the present invention, the term 'a glass filler or a blend of glass fillers' may be replaced by 'a blend of glass fillers'.

In yet another embodiment of the present invention the above described processes comprise adding of YbF₃ together with the monomer mixture and/or the glass filler or the blend of glass fillers.

The produced composite material was subjected to the Porosity Test, where distribution of porosity in the composite material was determined.

The one or more polymerizable monomers used in the embodiments are selected from the group comprising an acrylate or a methacrylate including but not limited to methyl acrylate, methyl methacrylate, ethyl acrylate, ethyl methacrylate, propyl acrylate, propyl methacrylate, isopropyl acrylate, isopropyl methacrylate, 2-hydroxyethyl acrylate, 2-hydroxyethyl methacrylate, hydroxypropyl acrylate, hydroxypropyl methacrylate, tetrahydrofurfuryl acrylate, tetrahydrofurfuryl methacrylate, glycidyl acrylate, glycidyl methacrylate, the diglycidyl methacrylate of bis-phenol A, glycerol mono-and di- acrylate, glycerol mono- and dimethacrylate, ethyleneglycol diacrylate, ethyleneglycol dimethacrylate, polyethyleneglycol diacrylate (where the number of repeating ethylene oxide units vary from 2 to 30), polyethyleneglycol dimethacrylate (where the number of repeating ethylene oxide units vary from 2 to 30 especially triethylene glycol dimethacrylate, neopentyl glycol diacrylate, neopentylglycol dimethacrylate, trimethylolpropane triacrylate, trimethylol propane trimethacrylate, mono-, di-, tri-, and tetra- acrylates and methacrylates of pentaerythritol and dipentaerythritol, 1,3-butanediol diacrylate, 1,3-butanediol dimethacrylate, 1,4-butanedioldiacrylate, 1,4-butanediol dimethacrylate, 1,6-hexane diol diacrylate, 1,6-hexanediol dimethacrylate, di-2-methacryloyloxethyl hexamethylene dicarbamate, di-2-methacryloyloxyethyl trimethylhexanethylene dicarbamate, di-2-methacryloyl oxyethyl dimethylbenzene dicarbamate, methylene-bis-2-methacryloxyethyl-4-cyclohexyl carbamate, di-2-methacryloxyethyl-dimethylcyclohexane dicarbamate, methylene-bis-2-methacryloxyethyl-4-cyclohexyl carbamate, di-1-methyl-2-methacryloxyethyl-trimethyl-hexamethylene dicarbamate, di-1-methyl-2-methacryloxyethyl-dimethylbenzene dicarbamate, di-1-methyl-2-methacryloxyethyl-dimethylcyclohexane dicarbamate, methylene-bis-1-methyl-2-methacryloxyethyl-4-cyclohexyl carbamate, di-1-chloromethyl-2-methacryloxyethyl-hexamethylene dicarbamate, di-1-chloromethyl-2-methacryloxyethyl-trimethylhexamethylene dicarbamate, di-1-chloromethyl-2-methacryloxyethyl-dimethylbenzene dicarbamate, di-1-chloromethyl-2-methacryloxyethyl-dimethylcyclohexane dicarbamate, methylene-bis-2-methacryloxyethyl-4-cyclohexyl carbamate, di-1-methyl-2-methacryloxyethyl-hexamethylene dicarbamate, di-1-methyl-2-methacryloxyethyl-trimethylhexamethylene dicarbamate, di-1-methyl-2-methacryloxyethyl-dimethylbenzene dicarbamate, di-1-methyl-2-methacryloxyethyl-dimethylcyclohexane dicarbamate, methylene-bis-1-methyl-2-methacryloxyethyl-4-cyclohexyl carbamate, di-1-chloromethyl-2-methacryloxyethyl-hexamethylene dicarbamate, di-1-chloromethyl-2-methacryloxyethyl-trimethylhexamethylene dicarbamate, di-1-chloromethyl-2-methacryloxyethyl-dimethylbenzene dicarbamate, di-1-chloromethyl-2-methacryloxyethyl-dimethylcyclohexane dicarbamate, methylene-bis-1-chloromethyl-2-methacryloxyethyl4-cyclohexyl carbamate, 2,2'-bis(4-methacryloxyphenyl)propane, 2,2'bis(4-acryloxyphenyl)propane, 2,2'-bis[4(2-hydroxy-3-methacryloxy-phenyl)]propane, 2,2'-bis[4(2-hydroxy-3-acryloxy-phenyl)propane, 2,2'-bis(4-methacryloxyethoxyphenyl)propane, 2,2'-bis(4-acryloxyethoxyphenyl)propane, 2,2'-bis(4-methacryloxypropoxyphenyl)propane, 2,2'-bis(4-acryloxypropoxyphenyl)propane, 2,2'-bis(4-methacryloxydiethoxyphenyl)propane, 2,2'-bis(4-acryloxydiethoxyphenyl)propane, 2,2'-bis[3(4-phenoxy)-2-hydroxypropane-1-methacrylate]propane, and 2,2'-bis[3(4-phenoxy)-2-hydroxypropane-1-acryalte]propane.

In the preferred embodiments of the present invention, the one or more polymerizable monomers are selected from the group comprising ethoxylated bisphenol dimethacrylate , triethylene glycol dimethacrylate, urethane dimethacrylate and Ormosil-Resin.

The polymerization stabilizer used in the embodiments of the present invention is selected from the group comprising butylated hydroxytoluene, hydroquinone monomethylether, tert.-butyl hydroquinone , tert.-butyl hydroxyanisole, 2,6-di-tert.-butyl-p-cresol, tetramethyl piperidine N-oxyl radical and galvanoxyl radical.

In the preferred embodiments of the present invention the polymerization stabilizer is butylated hydroxytoluene.

The polymerization initiator used in the embodiments of the present invention is a photoinitiator capable of promoting polymerization of the polymerizable groups on exposure to light of a suitable wavelength and intensity. The photoinitiator preferably is sufficiently shelf-stable and free of undesirable coloration to permit its storage and use under typical dental conditions. Visible light photoinitiators are preferred. Suitable visible light-induced and ultraviolet light-induced initiators include an alpha-diketone (e.g., camphorquinone) with or without additional hydrogen donors (such as sodium benzene sulfinate, amines and amino alcohols). The photoinitiator is present in an amount sufficient to provide the desired rate of photopolymerization.

Alternatively, a redox system can be used as a polymerization initiator. Redox type polymerization initiator systems of the present invention comprise but are not limited to the systems of organic peroxide/ amine compound or organic peroxide/ amine compound/ sulfinic acid salt, or inorganic peroxide/ amine compound/ sulfinic acid salt, wherein the organic peroxides may include but are not limited to benzoylperoxide, lauroyl peroxide, and cumene hydroperoxide, the inorganic peroxides may include but are not limited to potassium peroxosulphate, sodium peroxosulphate, the amines may include but are not limited to N-methylaniline, N,N-dimethylaniline, t-butyl-N,N-dimethylaniline, p-N,N-dimethyltoluidine and the sulfinic acid salts may include but are not limited to sodium p-toluenesulfinate, lithium p-toluenesulfinate sodium benzenesulfinate, and lithium benzenesulfinate.

In the preferred embodiments of the present invention, the initiator comprises camphorquinone and ethyl 4-(dimethylamino)benzoate.

The glass filler of the present invention may be a silanated glass filler.

The surface of the glass filler of the present invention may be modified prior to the mixing step. Accordingly, the surface modifying agent contains a modifying compound capable of reacting with surface atoms of the filler, thereby forming a covalent bond between the surface atoms of the filler and the modifying compound. Additionally, the modifying compound may contain one or more polymerizable double bonds reactive in the crosslinking reaction after the filler is modified. The modifying agent may contain one or more modifying compounds. Preferably, the modifying compound provides a polymerizable ligand capable of crosslinking which may be a compound of one of the following formulae (I), (II) and (III), or a hydrolysis product thereof

XᵣR₃₋ᵣSiL (I)

XᵣR₂₋ᵣSiL'L" (II)

XᵣSiL'L"L‴ (III)

wherein
X represents a hydrolyzable group;
R represents an alkyl, cycloalky, cycloalkylalkyl, aralkyl or aryl group, L, L', L", and L‴
   which may be the same or different represent independent from each other an organic group containing one or more polymerizable double bonds;
r is an integer of 1 to 3,
whereby the sum of X, R, L, L', L", and L'" is 4 for each of formula (I), (II), and (III).

Preferably, X is a halogen atom or OR¹ , wherein R¹ is an alkyl, cycloalky, cycloalkylalkyl, aralkyl or aryl group. More preferably, R or R¹ are independently an alkyl group.

In order to impart crosslinking capability to the organofunctional silicon compound, L, L', L", and L'" contain one or more polymerizable double bonds capable of taking part in a crosslinking reaction. In a preferred embodiment, L, L', L", and L'" may be selected from the group of allyl, (meth)acrylic ester groups, and (meth)acrylic amide groups.

An alkyl group may be straight-chain or branched CI-16 alkyl group, typically a C1-8 alkyl group. Examples for a C1-6 alkyl group can include linear or branched alkyl groups having 1 to 6 carbon atoms, preferably 1 to 4 carbon atoms, for example, methyl, ethyl, n-propyl, isopropyl, n-butyl, isobutyl, sec-butyl, tert-butyl, n-pentyl, isopentyl and n-hexyl. A cycloalkyl group may be a C3-16 cycloalkyl group. Examples of the cycloalkyl group can include those having 3 to 14 carbon atoms, for example, cyclopropyl, cyclobutyl, cyclopentyl and cyclohexyl. A cycloalkylalkyl group can include those having 4 to 22 carbon atoms. Examples for a cycloalkylalkyl group can include a combination of a linear or branched alkyl group having 1 to 6 carbon atoms and a cycloalkyl group having 3 to 14 carbon atoms. Examples of the cycloalkylalkyl group can for example, include methylcyclopropyl, methylcyclobutyl, methylcyclopentyl, methylcyclohexyl, ethylcyclopropyl, ethylcyclobutyl, ethylcyclopentyl, ethylcyclohexyl, propylcyclopropyl, propylcyclobutyl, propylcyclopentyl, propylcyclohexyl. An aralkyl group may be a C7-26 aralkyl group, typically a combination of a linear or branched alkyl group having 1 to 6 carbon atoms and an aryl group having 6 to 10 carbon atoms. Specific examples of an aralkyl group are a benzyl group or a phenylethyl group. An aryl group can include aryl groups having 6 to 10 carbon atoms. Examples of the aryl group are phenyl and naphtyl.

The CI-6 alkyl group and the C3-14 cycloalkyl group may optionally be substituted by one or more members of the group selected from a C1-4 alkyl group, C1-4 alkoxy group, a phenyl group, and a hydroxy group. Examples for a C1-4 alkyl group can include linear or branched alkyl groups having 1 to 4 carbon atoms, for example, methyl, ethyl, n-propyl, isopropyl, n-butyl, isobutyl, sec-butyl, tert-butyl. Examples for an C1-4 alkoxy group can include linear or branched alkoxy groups having 1 to 4 carbon atoms, for example, methoxy, ethoxy, n-propoxy, isopropoxy, n-butoxy, isobutoxy, sec-butoxy, and tert-butoxy.

Aryl groups may contain 1 to 3 substituents. Examples of such substituents can include halogen atoms, C1-4 alkyl groups, C1-4 alkoxy groups, C1-4 alkylthio groups, C1-4 alkylsulfonyl groups, carboxyl group, C2-5 alkoxycarbonyl groups, and C1-4 alkylamino groups. Here, illustrative of the halogen atoms can be fluorine, chlorine, bromine and iodine. The C1-4 alkyl groups are, for example, methyl, ethyl, n-propyl, isopropyl and n butyl. Illustrative of the C1-4 alkoxy groups are, for example, methoxy, ethoxy and propoxy. Illustrative of the C1-4 alkylthio groups are, for example, methylthio, ethylthio and propylthio. Illustrative of the C1-4 alkylsulfonyl groups are, for example, methylsulfonyl, ethylsulfonyl and propylsulfonyl. Illustrative of the C2-5 alkoxycarbonyl groups can be those having alkoxy groups each of which contains 1 to 4 carbon atoms, for example, methoxycarbonyl, ethoxy carbonyl and propoxycarbonyl. Illustrative of the C1-8 alkylamino groups can be those having one or two alkyl groups each of which contains 1 to 4 carbon atoms, for example, methylamino, dimethylamino, ethyl amino and propylamino. The alkyl moieties in these substituents may be linear, branched or cyclic.

It is an object of the present invention to provide a composite material produced by the above described processes.

It is an object of the present invention to provide a composite material comprising one or more polymerizable monomers, a polymerization initiator, a polymerization stabilizer and a glass filler or a blend of glass fillers, the composite material having a number of bubbles of size L lower than 20, a number of bubbles of size M lower than 100 and a number of bubbles of sizes M+L lower than 120, determined by a method defined in the Examples.

It is also an object of the present invention to provide a composite material comprising ethoxylated bisphenol dimethacrylate, triethylene glycol dimethacrylate , urethane dimethacrylate and Ormosil-Resin, camphorquinone and ethyl 4-(dimethylamino)benzoate, butylated hydroxytoluene, YbF₃, a blend of glass fillers and at least one pigment, the composite material having a number of bubbles of size L 12, a number of bubbles of size M 85 and a number of bubbles of sizes M+L 97, determined by a method defined in the Examples.

The composite material is for use in the dental industry, more particularly for use in producing a composite resin, a temporary sealing material, a resin cement, an adhesive or a fissure sealant.

### EXAMPLES

**EXAMPLE 1** describes a process for producing a composite material wherein a vacuum was applied into the mixing container before every single mixing step
**EXAMPLE 2** describes a process for producing a composite material wherein majority of the mixing steps were carried out under normal pressure
**EXAMPLE 3** describes a process of determining porosity of single composite materials and its results

### EXAMPLE 1

At 25°C in a 301 vessel of a planetary mixer to 3,5 kg of a monomer mixture comprising ethoxylated bisphenol dimethacrylate ,triethylene glycol dimethacrylate, urethane dimethacrylate and Ormosil-Resin, camphorquinone/ ethyl 4-(dimethylamino)benzoate, butylated hydroxytoluene, 0,6 kg of YbF₃ and 5,9 kg of a filler blend, containing 55 wt% of a silanated glass filler and 45 wt% of the SphereTEC filler, was added.

In the 1^{st} step the mixture was set under a vacuum of 700 ± 50 mbar before the mixing was carried out at 10 rpm for 3mins and at 15 rpm for 10 mins. In the 2^{nd} step 2,8 kg of the filler blend was added, the mixture was set under a vacuum of 700 ± 50 mbar before mixing was carried out at 10 rpm for 3mins and at 15 rpm for 10 mins. In the 3^{rd} step 1,2 kg of the filler blend was added, the mixture was set under a vacuum of 700 ± 50 mbar before mixing was carried out at 10 rpm for 3mins and at 15 rpm for 10 mins. In the 4^{th} step 0,9 kg of the filler blend was added, the mixture set under a vacuum of 700 ± 50 mbar before mixing was carried out at 15 rpm for 25 mins. In the 5^{th} step 0,7 kg of the filler blend was added, the mixture set under a vacuum of 700 ± 50 mbar before mixing was carried out at 15 rpm for 25 mins. In the 6^{th} step 0,4 kg of the filler blend was added, the mixture set under a vacuum of 700 ± 50 mbar before mixing was carried out at 15 rpm for 25 mins. In the 7^{th} and 8^{th} step the mixture set under a vacuum of 700 ± 50 mbar before mixing was carried out at 12 rpm for 120 mins. After that the mixture was stored at 50°C for 45 h before it was in the 9^{th} step at 48°C set under a vacuum of 220 ± 5 mbar before mixing was carried out at 8 rpm for 20 mins.

At 25°C to this mixture 61,7 g of pigments, 24,4 g of YbF₃ and 524 g of the filler blend, was added. In the 10^{th} step the mixture was set under a vacuum of 700 ± 50 mbar before mixing was carried out at 15 rpm for 30 mins. In the steps #11 to #13 the mixture was set under a vacuum of 700 ± 50 mbar before mixing was caried out at 15 rpm for 45 mins. In the 14^{th} step the mixture was set under a vacuum of 50 ± 5 mbar before mixing was carried out at 8 rpm for 15 mins. In the last step the mixture was set under a vacuum of 50 ± 5 mbar before finally the mixing was carried out a 5 rpm for 5 mins.

The paste was then automatically filled into small containers comprising approx. 0,3 g of paste. Out of these containers the specimen discs for the porosity test were prepared.

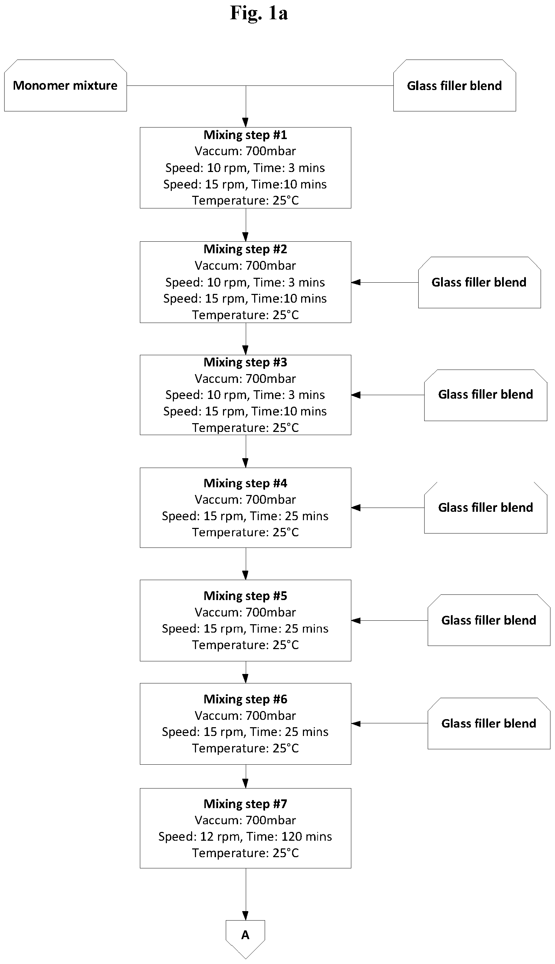

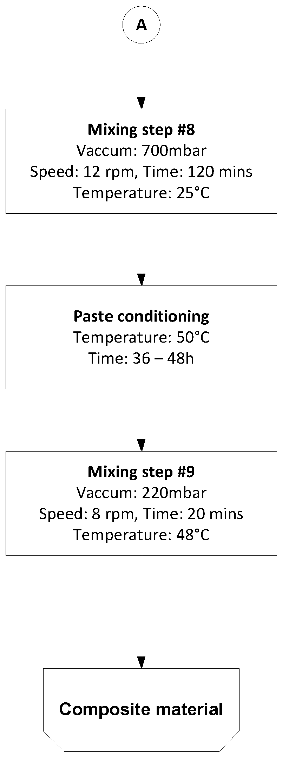

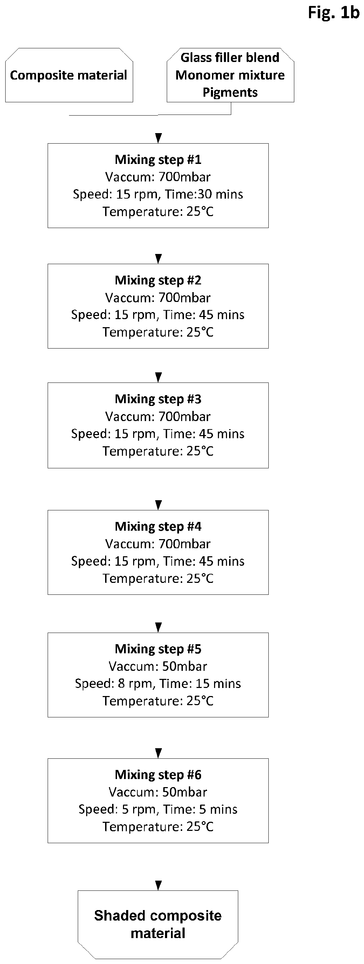

The process of the Example 1 is depicted in the Figs. 1a and 1b.

### EXAMPLE 2

At 25°C in a 301 vessel of a planetary mixer to 3,5 kg of a monomer mixture comprising ethoxylated bisphenol dimethacrylate ,triethylene glycol dimethacrylate, urethane dimethacrylate and Ormosil-Resin, camphorquinone/ ethyl 4-(dimethylamino)benzoate, butylated hydroxytoluene, 0,6 kg of YbF₃ and 5,9 kg of a filler blend, containing 55 wt% of a silanated glass filler and 45 wt% of the SphereTEC filler, was added.

In the 1^{st} step the mixing was carried out at 10 rpm for 3mins and at 15 rpm for 10 mins. In the 2^{nd} step 2,8 kg of the filler blend was added before mixing was carried out at 10 rpm for 3mins and at 15 rpm for 10 mins. In the 3^{rd} step 1,2 kg of the filler blend was added before mixing was carried out at 10 rpm for 3mins and at 15 rpm for 10 mins. In the 4^{th} step 0,9 kg of the filler blend was added before mixing was carried out at 15 rpm for 25 mins. In the 5^{th} step 0,7 kg of the filler blend was added before mixing was carried out at 15 rpm for 25 mins. In the 6^{th} step 0,4 kg of the filler blend was added before mixing was carried out at 15 rpm for 25 mins. In the 7^{th} and 8^{th} step mixing was carried out at 12 rpm for 120 mins. After that the mixture was stored at 50°C for 45 h before it was in the 9^{th} step at 48°C set under a vacuum of 220 ± 5 mbar before mixing was carried out at 8 rpm for 20 mins.

At 25°C to this mixture 61,5 g of pigments, 24,6 g of YbF₃ and 530 g of the filler blend, was added. In the 10^{th} step the mixture was mixed at 15 rpm for 30 mins. In the steps #11 to #13 mixing was caried out at 15 rpm for 45 mins. In the 14^{th} step the mixture was set under a vacuum of 50 ± 5 mbar before mixing was carried out at 8 rpm for 15 mins. In the last step the mixture was set under a vacuum of 50 ± 5 mbar before finally the mixing was carried out a 5 rpm for 5 mins.

The paste was then automatically filled into small containers comprising approx. 0,3 g of paste. Out of these containers the specimen discs for the porosity test were prepared.

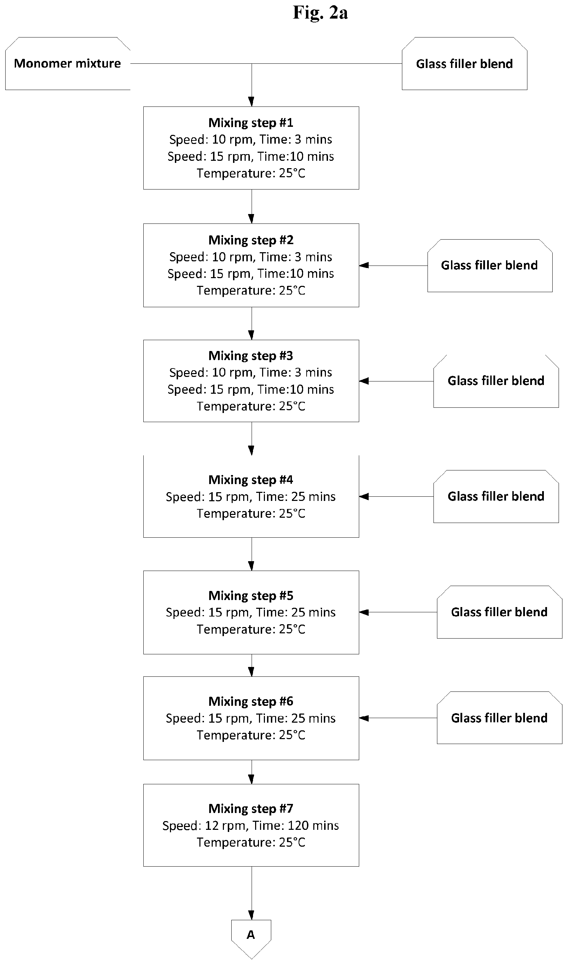

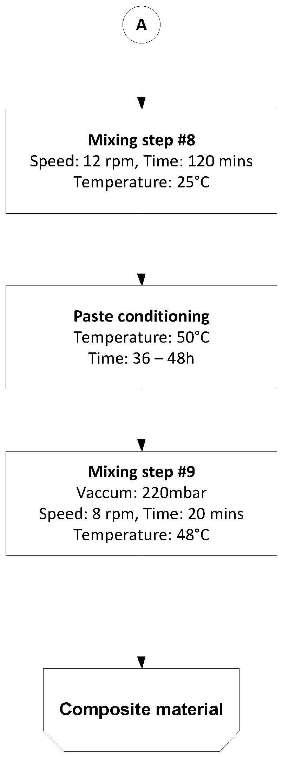

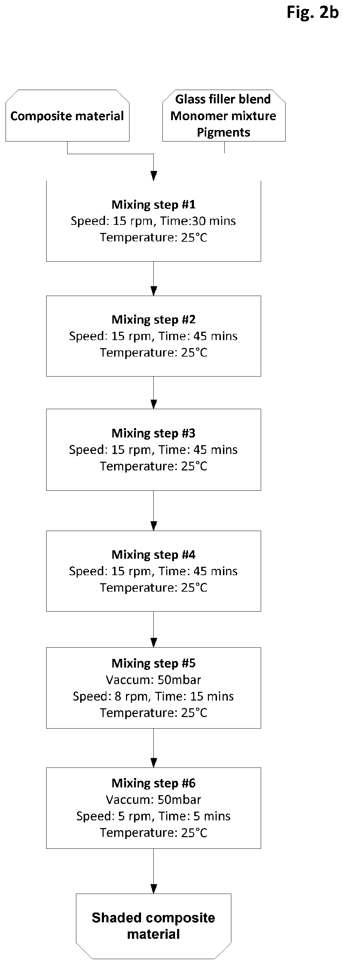

The process of the Example 2 is depicted in the Figs. 2a and 2b

### EXAMPLE 3

### 1. Sample preparation

Composite material is placed in the middle of a Teflon mold with the thickness of 0.55 +- 0.05mm and covered on the bottom and the top with a Mylar foil. The material is then pressed with the help of a glass plate until the mold is completely filled. The test specimen, including foils, is carefully placed between glass slides, fixed with clamps and placed vertically in a holder in the LicuLite curing experiment. Each side of the specimen is cured for 1 minute and then the cured disc is removed from the mold.

### 2. Analysis of porosity

The sample disc is placed under a microscope and a photo is taken using the imaging software "VLC media player" with driver "Blackmagic Design Desktop video 9.5.3" or the imaging software "Grab IT".

After that the air bubbles depending on their size are automatically counted using the software "dhs Bilddatenbank".

The specification of the air bubbles are as follows:

| **Size** | **Specification (µm)** | **Evaluation of criticality** |
|---|---|---|
| XS | 0-30 | noncritical |
| S | 30-70 | noncritical |
| M | 70 - 140 | semi-critical |
| L | 140 - 1000 | critical |

**Tabl: Porosity Test, number of air bubbles per specimen disc**

| **Paste** | **M size** | **L size** | **Sum M + L** |
|---|---|---|---|
| Composite material of Example 1 | 85 | 12 | 97 |
| Composite material of Example 2 | 261 | 57 | 318 |

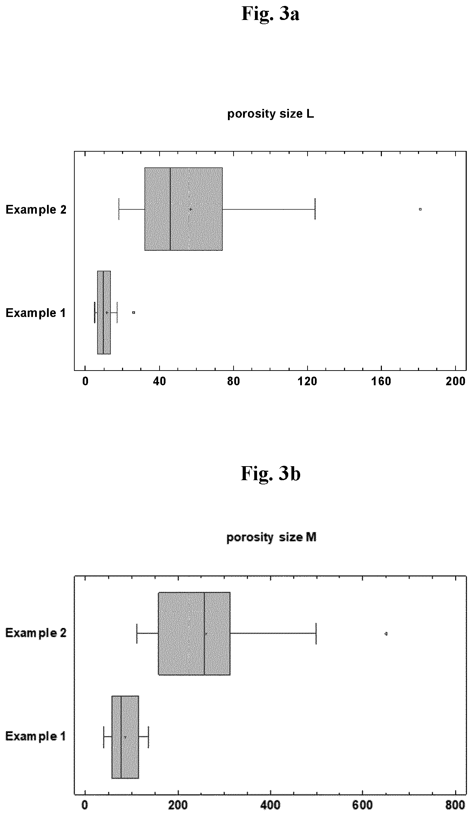

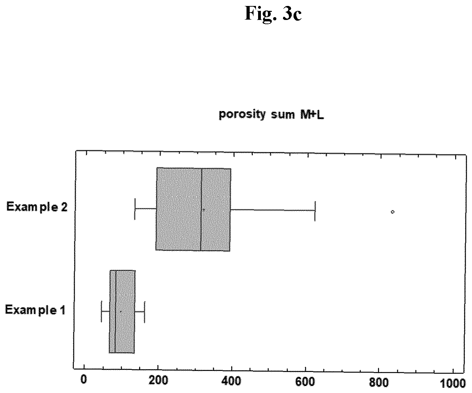

Figs. 3a, 3b and 3c depict the results of the Example 3.

**In other words, the results of the Porosity Test clearly and unambiguously demonstrate that the composite material produced by a process wherein a vacuum is set in the mixing container before every single mixing step starts shows much lower level of porosity when compared with the composite material produced by a process of the same steps and conditions, but wherein a vacuum was set just before the last step or before the last two steps.**

## Claims

1. A process for producing a composite material comprising the steps of:
a. Charging a monomer mixture into the mixing container
b. Charging a part of the glass filler or of the blend of glass fillers into the mixing container to create a mixture, setting the mixture under a vacuum and mixing the mixture
c. Charging a part of the glass filler or of the blend of glass fillers into the mixing container to create a mixture, setting the mixture under a vacuum and mixing the mixture
d. Setting the mixture under a vacuum and mixing it to create a paste
e. Conditioning the paste
f. Setting the paste under a vacuum and mixing the paste to produce a composite material and optionally
g. Adding at least one pigment and optionally
a glass filler or a blend of glass fillers and/or a monomer mixture to the composite material to create a mixture, setting the mixture under a vacuum and mixing the mixture to produce a composite material.

2. A process according to claim 1, wherein :
- the vacuum of steps b., c. and d. is of 650-800 mbar,
- the mixing of step b. is carried out at the speed of 8-12rpm for 2-5 minutes and then at the speed of 13-16 rpm for 8-12 minutes at the temperature of 23-27°C, whereby the step can be carried out 1-5x
- the mixing of step c. is carried out at the speed of 12-16 rpm for 22-27 minutes at the temperature of 23-27°C, whereby the step can be carried out 1-4x
- the mixing of step d. is carried out at the speed of 10-14rpm for 110-130 minutes at the temperature of 23-27°C, whereby this step can be carried out 1-3x
- the conditioning of step e. is carried out at the temperature of 45-55°C for 36-50 hours under anaerobic conditions
- the vacuum of step f. is a vacuum of 210-230 mbar and the mixing of step f. is carried out at the speed of 6-10rpm for 18-22 minutes at the temperature of 46-48°C
- the mixing of step g. is carried out in at least three steps at the temperature of 23-27°C at the speed of 5-15rpm, whereby each of the steps lasts from 5 to 45 minutes and whereby a vacuum of 45-800 mbar is set before every single mixing step starts.

3. The process of claim 2, comprising the following steps:
a. Charging the monomer mixture into the mixing container
b. Charging the first part of the glass filler or of the blend of glass fillers into the mixing container to create a mixture, setting the mixture under a vacuum of 700mbar and mixing it at the speed of 10rpm for 3 minutes and then at the speed of 15 rpm for 10mins at the temperature of 25°C.
c. Charging the second part of the glass filler or of the blend of glass fillers into the mixing container to create a mixture, setting the mixture under a vacuum of 700mbar and mixing it at the speed of 10rpm for 3 minutes and then at the speed of 15 rpm for 10mins at the temperature of 25°C.
d. Charging the third part of the of glass filler or of the blend of glass fillers into a mixing container, to create a mixture, setting the mixture under a vacuum of 700mbar and mixing it at the speed of 10rpm for 3 minutes and then at the speed of 15 rpm for 10mins at the temperature of 25 °C.
e. Charging the fourth part of the glass filler or of the blend of glass fillers into a mixing container to create a mixture, setting the mixture under a vacuum of 700mbar and mixing it at the speed of 15rpm for 25 minutes at the temperature of 25°C
f. Charging the fifth part of the glass filler or of the blend of glass fillers into a mixing container to create a mixture, setting the mixture under a vacuum of 700mbar and mixing it at the speed of 15rpm for 25 minutes at the temperature of 25°C.
g. Charging the sixth part of the glass filler or of the blend of glass fillers into a mixing container to create a mixture, setting the mixture under a vacuum of 700mbar and mixing it at the speed of 15rpm for 25 minutes at the temperature of 25 °C to create a mixture
h. Setting the mixture under a vacuum of 700mbar and mixing it at the speed of 12 rpm for 120 minutes at the temperature of 25 °C to create a mixture
i. Setting the mixture under a vacuum of 700 mbar and mixing it at the speed of 12 rpm for 120 minutes at the temperature of 25 °C to create a paste.
j. Conditioning the paste at the temperature of 50 °C for 36-48 hours under anaerobic conditions.
k. Setting the paste under a vacuum of 220 mbar into the mixing container and mixing it at the speed of 8rpm for 20 minutes at the temperature of 48°C to produce a composite material and optionally
l. Adding at least one pigment and optionally
a glass filler or a blend of glass fillers and/or a monomer mixture to the composite material to create a mixture, setting the mixture under a vacuum of 700mbar and mixin it at the speed of 15 rpm for 30 minutes at the temperature of 25°C.
m. Setting the mixture under a vacuum of 700 mbar and mixing it at the speed of 15 rpm for 45 minutes at the temperature of 25°C
n. Setting the mixture under a vacuum of 700 mbar and mixing it at the speed of 15 rpm for 45 minutes at the temperature of 25°C
o. Setting the mixture under a vacuum of 700 mbar and mixing it at the speed of 15 rpm for 45 minutes at the temperature of 25°C
p. Setting the mixture under a vacuum of 50 mbar and mixing it at the speed of 8 rpm for 15 minutes at the temperature of 25°C
q. Setting the mixture under a vacuum of 50 mbar and mixing it at the speed of 5 rpm for 5 minutes at the temperature of 25°C to produce a composite material.

4. A process according to any of the preceding claims, wherein the monomer mixture comprises one or more polymerizable monomers, a polymerization initiator and a polymerization stabilizer.

5. A process according to any of the preceding claims, wherein the one or more polymerizable monomers are selected from the group comprising acrylates and methacrylates including, but not limited to methyl acrylate, methyl methacrylate, ethyl acrylate, ethyl methacrylate, propyl acrylate, propyl methacrylate, isopropyl acrylate, isopropyl methacrylate, 2-hydroxyethyl acrylate, 2-hydroxyethyl methacrylate, hydroxypropyl acrylate, hydroxypropyl methacrylate, tetrahydrofurfuryl acrylate, tetrahydrofurfuryl methacrylate, glycidyl acrylate, glycidyl methacrylate, the diglycidyl methacrylate of bis-phenol A, glycerol mono-and di- acrylate, glycerol mono- and di- methacrylate, ethyleneglycol diacrylate, ethyleneglycol dimethacrylate, polyethyleneglycol diacrylate (where the number of repeating ethylene oxide units vary from 2 to 30), polyethyleneglycol dimethacrylate (where the number of repeating ethylene oxide units vary from 2 to 30 especially triethylene glycol dimethacrylate, neopentyl glycol diacrylate, neopentylglycol dimethacrylate, trimethylolpropane triacrylate, trimethylol propane trimethacrylate, mono-, di-, tri-, and tetraacrylates and methacrylates of pentaerythritol and dipentaerythritol, 1,3-butanediol diacrylate, 1,3-butanediol dimethacrylate, 1,4-butanedioldiacrylate, 1,4-butanediol dimethacrylate, 1,6-hexane diol diacrylate, 1,6-hexanediol dimethacrylate, di-2-methacryloyloxethyl hexamethylene dicarbamate, di-2-methacryloyloxyethyl trimethylhexanethylene dicarbamate, di-2-methacryloyl oxyethyl dimethylbenzene dicarbamate, methylene-bis-2-methacryloxyethyl-4-cyclohexyl carbamate, di-2-methacryloxyethyl-dimethylcyclohexane dicarbamate, methylene-bis-2-methacryloxyethyl-4-cyclohexyl carbamate, di-1-methyl-2-methacryloxyethyl-trimethyl-hexamethylene dicarbamate, di-1-methyl-2-methacryloxyethyl-dimethylbenzene dicarbamate, di-1-methyl-2-methacryloxyethyl-dimethylcyclohexane dicarbamate, methylene-bis-1-methyl-2-methacryloxyethyl-4-cyclohexyl carbamate, di-1-chloromethyl-2-methacryloxyethyl-hexamethylene dicarbamate, di-1-chloromethyl-2-methacryloxyethyl-trimethylhexamethylene dicarbamate, di-1-chloromethyl-2-methacryloxyethyl-dimethylbenzene dicarbamate, di-1-chloromethyl-2-methacryloxyethyl-dimethylcyclohexane dicarbamate, methylene-bis-2-methacryloxyethyl-4-cyclohexyl carbamate, di-1-methyl-2-methacryloxyethyl-hexamethylene dicarbamate, di-1-methyl-2-methacryloxyethyl-trimethylhexamethylene dicarbamate, di-1-methyl-2-methacryloxyethyl-dimethylbenzene dicarbamate, di-1-methyl-2-metha-cryloxyethyl-dimethylcyclohexane dicarbamate, methylene-bis-1-methyl-2-methacryloxyethyl-4-cyclohexyl carbamate, di-1-chloromethyl-2-methacryloxyethyl-hexamethylene dicarbamate, di-1-chloromethyl-2-methacryloxyethyl-trimethylhexamethylene dicarbamate, di-1-chloromethyl-2-methacryloxyethyl-dimethylbenzene dicarbamate, di-1-chloromethyl-2-methacryloxyethyl-dimethylcyclohexane dicarbamate, methylene-bis-1-chloromethyl-2-methacryloxyethyl4-cyclohexyl carbamate, 2,2'-bis(4-methacryloxyphenyl)propane, 2,2'bis(4-acryloxyphenyl)propane, 2,2'-bis[4(2-hydroxy-3-methacryloxy-phenyl)]propane, 2,2'-bis[4(2-hydroxy-3-acryloxy-phenyl)propane, 2,2'-bis(4-methacryloxyethoxyphenyl)propane, 2,2'-bis(4-acryloxyethoxyphenyl)propane, 2,2'-bis(4-methacryloxypropoxyphenyl)propane, 2,2'-bis(4-acryloxypropoxyphenyl)propane,2,2'-bis(4-ethacryloxydiethoxyphenyl)propane, 2,2'-bis(4-acryloxydiethoxyphenyl)propane, 2,2'-bis[3(4-phenoxy)-2-hydroxypropane-1-methacrylate]propane, and 2,2'-bis[3(4-phenoxy)-2-hydroxypropane-1-acryalte]propane.

6. A process according to any of the preceding claims, wherein the one or more polymerizable monomers are selected from the group comprising ethoxylated bisphenol dimethacrylate , triethylene glycol dimethacrylate, urethane dimethacrylate and Ormosil-Resin.

7. A process according to any of the preceding claims, wherein the polymerization initiator is one or more compounds selected from the group comprising visible light and ultraviolet light-induced initiators including alpha-diketones with or without at least one additional hydrogen donor or the polymerization initiator is a redox system.

8. A process according to any of the preceding claims, wherein the polymerization initiator comprises camphorquinone and ethyl 4-(dimethylamino)benzoate.

9. A process of claim 7, wherein the redox system comprises the systems of organic peroxide/amine compound or organic peroxide/amine compound/ sulfinic acid salt or inorganic peroxide/amine compound/sulfinic acid salt.

10. A process according to any of the preceding claims, wherein the polymerization stabilizer is selected from the group comprising butylated hydroxytoluene, hydroquinone monomethylether, tert.-butyl hydroquinone , tert.-butyl hydroxyanisole, 2,6-di-tert.-butyl-p-cresol, tetramethyl piperidine N-oxyl radical and galvanoxyl radical.

11. A process according to any of the preceding claims, wherein the glass filler is a silanated glass filler.

12. A process of claims 1-11, wherein the mixing container is a planetary mixer or a centrifugal mixer.

13. A composite material comprising one or more polymerizable monomers, a polymerization initiator, a polymerization stabilizer and a glass filler or a blend of glass fillers, having a number of bubbles of size L lower than 20, a number of bubbles of size M lower than 100 and a number of bubbles of sizes M+L lower than 120, determined by a method defined in the description.

14. A composite material of claim 13 for use in the dental industry.

15. A composite material of claim 13 for use in producing a composite resin, a temporary sealing material, a resin cement, an adhesive or a fissure sealant.

## Amended claims

### Amended claims in accordance with Rule 137(2) EPC.

1. A process for producing a composite material comprising the following steps in the following order :
a. Charging a monomer mixture into the mixing container
b. Charging a part of the glass filler or of the blend of glass fillers into the mixing container to create a mixture, setting the mixture under a vacuum and mixing the mixture
c. Charging another part of the glass filler or of the blend of glass fillers into the mixing container to create a mixture, setting the mixture under a vacuum and mixing the mixture
d. Setting the mixture under a vacuum and mixing it to create a paste
e. Storing the paste at an elevated temperature
f. Setting the paste under a vacuum and mixing the paste to produce a composite material and optionally
g. Adding at least one pigment and optionally
a glass filler or a blend of glass fillers and/or a monomer mixture to the composite material to create a mixture, setting the mixture under a vacuum and mixing the mixture to produce a composite material,
wherein the monomer mixture comprises one or more polymerizable monomers, a polymerization initiator and a polymerization stabilizer, wherein the one or more polymerizable monomers are selected from the group comprising acrylates and methacrylates.

2. The process according to claim 1, wherein :
- the vacuum of steps b., c. and d. is of 650-800 mbar,
- the mixing of step b. is carried out at the speed of 8-12rpm for 2-5 minutes and then at the speed of 13-16 rpm for 8-12 minutes at the temperature of 23-27°C, whereby the step can be carried out 1-5x
- the mixing of step c. is carried out at the speed of 12-16 rpm for 22-27 minutes at the temperature of 23-27°C, whereby the step can be carried out 1-4x
- the mixing of step d. is carried out at the speed of 10-14rpm for 110-130 minutes at the temperature of 23-27°C, whereby this step can be carried out 1-3x
- the storing of step e. is carried out at the temperature of 45-55°C for 36-50 hours under anaerobic conditions
- the vacuum of step f. is a vacuum of 210-230 mbar and the mixing of step f. is carried out at the speed of 6-1 0rpm for 18-22 minutes at the temperature of 46-48°C
- the mixing of step g. is carried out in at least three steps at the temperature of 23-27°C at the speed of 5-15rpm, whereby each of the steps lasts from 5 to 45 minutes and whereby a vacuum of 45-800 mbar is set before every single mixing step starts.

3. The process of claim 2, comprising the following steps:
a. Charging the monomer mixture into the mixing container
b. Charging the first part of the glass filler or of the blend of glass fillers into the mixing container to create a mixture, setting the mixture under a vacuum of 700mbar and mixing it at the speed of 10rpm for 3 minutes and then at the speed of 15 rpm for 10mins at the temperature of 25°C.
c. Charging the second part of the glass filler or of the blend of glass fillers into the mixing container to create a mixture, setting the mixture under a vacuum of 700mbar and mixing it at the speed of 10rpm for 3 minutes and then at the speed of 15 rpm for 10mins at the temperature of 25°C.
d. Charging the third part of the of glass filler or of the blend of glass fillers into a mixing container, to create a mixture, setting the mixture under a vacuum of 700mbar and mixing it at the speed of 10rpm for 3 minutes and then at the speed of 15 rpm for 10mins at the temperature of 25 °C.
e. Charging the fourth part of the glass filler or of the blend of glass fillers into a mixing container to create a mixture, setting the mixture under a vacuum of 700mbar and mixing it at the speed of 15rpm for 25 minutes at the temperature of 25°C
f. Charging the fifth part of the glass filler or of the blend of glass fillers into a mixing container to create a mixture, setting the mixture under a vacuum of 700mbar and mixing it at the speed of 15rpm for 25 minutes at the temperature of 25°C.
g. Charging the sixth part of the glass filler or of the blend of glass fillers into a mixing container to create a mixture, setting the mixture under a vacuum of 700mbar and mixing it at the speed of 15rpm for 25 minutes at the temperature of 25 °C to create a mixture
h. Setting the mixture under a vacuum of 700mbar and mixing it at the speed of 12 rpm for 120 minutes at the temperature of 25 °C to create a mixture
i. Setting the mixture under a vacuum of 700 mbar and mixing it at the speed of 12 rpm for 120 minutes at the temperature of 25 °C to create a paste.
j. Storing the paste at the temperature of 50 °C for 36-48 hours under anaerobic conditions.
k. Setting the paste under a vacuum of 220 mbar into the mixing container and mixing it at the speed of 8rpm for 20 minutes at the temperature of 48°C to produce a composite material and optionally
l. Adding at least one pigment and optionally
a glass filler or a blend of glass fillers and/or a monomer mixture to the composite material to create a mixture, setting the mixture under a vacuum of 700mbar and mixin it at the speed of 15 rpm for 30 minutes at the temperature of 25°C.
m. Setting the mixture under a vacuum of 700 mbar and mixing it at the speed of 15 rpm for 45 minutes at the temperature of 25°C
n. Setting the mixture under a vacuum of 700 mbar and mixing it at the speed of 15 rpm for 45 minutes at the temperature of 25°C
o. Setting the mixture under a vacuum of 700 mbar and mixing it at the speed of 15 rpm for 45 minutes at the temperature of 25°C
p. Setting the mixture under a vacuum of 50 mbar and mixing it at the speed of 8 rpm for 15 minutes at the temperature of 25°C
q. Setting the mixture under a vacuum of 50 mbar and mixing it at the speed of 5 rpm for 5 minutes at the temperature of 25°C to produce a composite material.

4. The process according to any of the preceding claims, wherein the polymerization initiator is one or more compounds selected from the group comprising visible light and ultraviolet light-induced initiators including alpha-diketones with or without at least one additional hydrogen donor or the polymerization initiator is a redox system with the proviso that the photoinitiator is not a system of organic peroxide/amine compound.

5. The process according to any of the preceding claims, wherein the polymerization initiator comprises camphorquinone and ethyl 4-(dimethylamino)benzoate.

6. The process according to any of the preceding claims, wherein the polymerization stabilizer is selected from the group comprising butylated hydroxytoluene, hydroquinone monomethylether, tert.-butyl hydroquinone , tert.-butyl hydroxyanisole, 2,6-di-tert.-butyl-p-cresol, tetramethyl piperidine N-oxyl radical and galvanoxyl radical.

7. The process according to any of the preceding claims, wherein the glass filler is a silanated glass filler.

8. The process of claims 1-7, wherein the mixing container is a planetary mixer or a centrifugal mixer.

9. A composite material comprising one or more polymerizable monomers, a polymerization initiator, a polymerization stabilizer and a glass filler or a blend of glass fillers, having a number of bubbles of size L lower than 20, a number of bubbles of size M lower than 100 and a number of bubbles of sizes M+L lower than 120,, wherein size M is of 70-140 µm and size L of 140-1000 µm.

10. The composite material of claim 9 for use in the dental industry.

11. The composite material of claim 9 for use in producing a composite resin, a temporary sealing material, a resin cement, an adhesive or a fissure sealant.
